# EUROPEAN PATENT APPLICATION

(11) **EP 4 682 897 A1**
(43) Date of publication of application: **21.01.2026**
(21) Application number: 25189991.0
(22) Date of filing: 16.07.2025
(51) Int. Cl.: G16H 15/00, G16H 20/40, G16H 30/40, G16H 40/63, G16H 70/20, G16H 80/00, G06N 3/08

(54) **MEDICAL INFORMATION PROCESSING APPARATUS AND MEDICAL INFORMATION PROCESSING METHOD**

(30) Priority: 17.07.2024 US 202418775626
(71) Applicant: Canon Medical Systems Corporation, Tochigi 324-0036 (JP)
(72) Inventor: RAZETO, Marco, Edinburgh, EH6 5NP (GB); HOOGENDOORN, Corne, Edinburgh, EH6 5NP (GB)
(74) Representative: Marks & Clerk LLP

(57) **Abstract**

A medical information processing apparatus (100) according to an embodiment includes processing circuitry (115). The processing circuitry is configured to: obtain a plurality of pieces of data collected, the plurality of pieces of data belonging to a plurality of data modalities (220a-220e),linking with a common timeline that is common to times at which the plurality of pieces of data are collected; for each of the plurality of data modalities (220a-220e), process data of each of the plurality of data modalities (220a-220e) to generate one or more labels occurring at a time on the common timeline and identify an event indicated by each of the processed plurality of pieces of data; and process the labels for each of the identified events based on the relative time of occurrence of the events to output information indicative of a further medical event.

## Description

### FIELD

The present disclosure relates to a medical information processing apparatus and a medical information processing method.

### BACKGROUND

A clinical procedure may involve a number of events that take place throughout the procedure. For example, a surgical procedure comprises a number of stages, such as the administration of suitable injections - **e.g.** anaesthetic - the insertion of an instrument - **e.g.** an endoscope - a cleaning phase, a dissection phase, a suturing phase. Additional events that may be identified throughout a clinical procedure, include all medical staff becoming present or a guidewire reaching a target. In order to provide guidance in real time or provide records relating to a procedure for future use, it may be desirable to identify the occurrence of such events that have taken place during the clinical procedure.
One way of identifying the occurrence of events is for a clinician to manually provide identification of events during the procedure based, for example, on the basis of a video recording of the procedure. However, the manual labelling of video data may consume a significant amount of the clinician's time that could otherwise be directed towards additional endeavours. Furthermore, the type of event that may be identified on the basis of video data alone may be quite limited. Another proposed approach may be to subject the video data to processing by an automated model. However, the type of events that may be identified based on the video data may be quite limited. Furthermore, there is the potential for inaccuracy in the evaluation of the video data.

### SUMMARY OF THE INVENTION

A medical information processing apparatus includes processing circuitry configured to: obtain a plurality of pieces of data collected during a clinical procedure, the plurality of pieces of data belonging to a plurality of data modalities, at least one of the plurality of data modalities being an imaging data type and any other of the plurality of data modalities than the imaging data type being a further data type different from imaging data, wherein the plurality of pieces of data is provided, linking with a common timeline that is common to times at which the plurality of pieces of data are collected; for each of the plurality of data modalities, process data of each of the plurality of data modalities to generate one or more labels occurring at a time on the common timeline and identify an event indicated by each of the processed plurality of pieces of data; and process the labels for each of the identified events based on the relative time of occurrence of the events to output information indicative of a further medical event.

For each of the plurality of data modalities, the step of processing the labels for each of the identified events may include providing the labels as inputs to a machine learning model to obtain information indicative of the further medical event.

For each of the data modalities, the processing of the data of each of the plurality of data modalities and the generation of the label may be performed in real time when one of the plurality of pieces of data is obtained. Providing the labels as inputs to the machine learning model may include, for each of the labels, upon the generation of the respective label: providing the respective label as an input to the machine learning model.

The machine learning model may be a recurrent neural network.

The processing circuitry may be configured to provide each of the labels as inputs to the machine learning model in an order in which the corresponding identified events occurred in the common timeline.

The processing circuitry may be configured to: for each of the identified events, output time information indicating a time in the common timeline at which the respective identified event occurred; and process the time information for the identified events to determine a time associated with the further medical event.

The imaging data may include at least one of: video data; X-ray image data; and ultrasonic image data.

The plurality of data modalities may include at least one of: video data; audio data; X-ray image data; ultrasonic image data; radio frequency tag data; and vital data.

For one or more of the plurality of data modalities: the processing of the data of each of the plurality of data modalities to identify the event occurring during the procedure may include providing the data of each of the data modalities to a further machine learning model to derive the label of the respective identified event.

One or more of the plurality of data modalities may include the imaging data. For the imaging data, the respective further machine learning model used to derive the label of the respective identified event may include a convolutional neural network.

One or more of the plurality of data modalities may include audio data. For the audio data, the respective further machine learning model used to derive the label of the respective identified event may include a speech recognition model configured to derive text representing the audio data.

The processing circuitry may be configured to process the text using a natural language understanding model to derive the label identifying the event.

The processing circuitry may be configured to control a display to display on the display visual information indicative of the further medical event and associated time information indicating when, in the clinical procedure, the further medical event took place.

The processing circuitry may be configured to execute an application corresponding to the further medical event in accordance with an output of information indicative of the further medical event.

A medical information processing method is performed by a medical information processing apparatus. The method includes: obtaining a plurality of pieces of data collected during a clinical procedure, the plurality of pieces of data belonging to a plurality of data modalities, at least one of the plurality of data modalities being an imaging data type and any other of the plurality of data modalities than the imaging data type being a further data type different from imaging data, wherein the plurality of pieces of data is provided, linking with a common timeline that is common to times at which the plurality of pieces of data are collected; for each of the plurality of data modalities, processing data of each of the plurality of data modalities to generate one or more labels occurring at a time on the common timeline and identifying an event indicated by each of the processed plurality of pieces of data; and processing the labels for each of the identified events based on the relative time of occurrence of the events to output information indicative of a further medical event.

### BRIEF DESCRIPTION OF THE DRAWINGS

Some embodiments of the disclosure will now be described, by way of example only and with reference to the accompanying drawings, in which:
Figure 1A illustrates a data processing apparatus according to embodiments;
Figure 1B illustrates a further example data processing apparatus that may be used for training one or more machine learning models;
Figure 2 illustrates a apparatus comprising the data processing in communication with a plurality of devices for collecting data belonging to different modalities;
Figure 3 illustrates a plurality of modules, each for processing data belonging to different channel to derive labels for sub-events;
Figure 4 illustrates the components of a module for processing a stream of data belonging to a channel;
Figure 5 illustrates an example of the use of data belonging to three different channels for deriving sub-event labels and an event label;
Figure 6 illustrates an example of a number of events that may be identified based on data belonging to different channels;
Figure 7 illustrates a neural network according to embodiments;
Figure 8A illustrates a first part of an example convolutional neural network for classifying frames of a video;
Figure 8B illustrates a second part of an example convolutional neural network for classifying frames of a video;
Figure 9A illustrates an example of a recurrent neural network (RNN);
Figure 9B illustrates an example of further example of a recurrent neural network (RNN);
Figure 10 illustrates an example of the training of a 3D convolutional neural network and an example of the training of a recurrent neural;
Figure 11 illustrates an example of the training a machine learning model to classify events based on a number of sub-event labels;
Figure 12 illustrates part of a training data set comprising a number of event labels and associated sub-event labels;
Figure 13 illustrates a method according to example embodiments; and
Figure 14 which illustrates an example of content that may be displayed on a user interface of device during or following a clinical procedure.

### DETAILED DESCRIPTION

During a clinical procedure data belonging to one or more modalities may be available for identifying the occurrence of particular events taking place as part of the procedure. Two common data modalities include video and audio, for example, but additional data modalities that may be available depend upon the type of procedure. For example, during a medical procedure, types of data in addition to video and audio that may be available could include imaging data derived from fluoroscopy, imaging data derived from another scanning technique, data obtained from a radio frequency tag, heart rate data, blood pressure data, temperature data, etc.

According to certain embodiments, there is provided a medical information processing apparatus comprising processing circuitry. The processing circuitry is configured to: obtain a plurality of pieces of data collected during a clinical procedure, the plurality of pieces of data belonging to a plurality of data modalities, at least one of the plurality of data modalities being an imaging data type and the plurality of data modalities including a further data type different from imaging data, wherein the plurality of pieces of data is provided, linking with a common timeline that is common to times at which the plurality of pieces of data are collected; for each of the plurality of data modalities (220a-220e), process data of each of the plurality of data modalities (220a-220e) to generate one or more labels occurring at a time on the common timeline and identify an event indicated by each of the processed plurality of pieces of data; and process the labels for each of the identified events based on the relative time of occurrence of the events to output information indicative of a further medical event.

The plurality of pieces of data belonging to a plurality of data modalities means herein that each of the plurality of pieces of data belongs to any of the plurality of data modalities. Processing the labels for each of the identified events based on the relative time of occurrence of the events to output information indicative of a further medical event means outputting an event label, in other words, a medical event from a relationship between the relative times of occurrence of a plurality of sub-events.

By monitoring and processing data belonging to different modalities sub-events may be identified in the data of a number of those modalities. Labels of those sub-event may then used by to derive classifications of higher level events based on the time at which the labelled events occurred. As a result, identification of higher levels events may be performed with greater accuracy. The classifications of higher levels may then be tagged and added to the overall timeline. This allows automatic reporting and analysis of the procedure workflow, which may be performed in real-time during the procedure or may be performed following the procedure to produce a labelled summary of a past procedure. The identified higher-level events are presented as salient moments in a summarized procedure.

The data belonging to the different data modalities is provided with reference to a common timeline. In other words, each item of the data (e.g. a frame of a video, a segment of audio data, and an RF tag measurement) may be associated with a particular time during the clinical procedure. The data processing apparatus receives this time information indicating the particular time of each item of data along with the data itself. The time information may be used to derive timestamps associated with the sub-events. The timestamps may then be used to derive time information for the higher-level events, which may be placed at an appropriate point on the timeline.

According to certain embodiments, there is provided a method comprising: receiving data collected during a clinical procedure, the data belonging to a plurality of data modalities, at least one of the data modalities being an imaging data type and a further of the data modalities being an additional data type other than imaging data, wherein the data is provided with reference to a common timeline over which the data is collected; for each of the plurality of data modalities, processing data of the respective data modality to generate a label identifying an event occurring at a time on the common timeline and indicated by the processed data; and processing the labels for each of the identified events based on the times of occurrence of the events to obtain an output indicative of a further medical event.

According to certain embodiments, there is provided a computer program comprising computer readable instructions, which when executed by at least one processor, causes the at least one processor to perform a method comprising: receiving data collected during a clinical procedure, the data belonging to a plurality of data modalities, at least one of the data modalities being an imaging data type and a further of the data modalities being an additional data type other than imaging data, wherein the data is provided with reference to a common timeline over which the data is collected; for each of the plurality of data modalities, processing data of the respective data modality to generate a label identifying an event occurring at a time on the common timeline and indicated by the processed data; and processing the labels for each of the identified events based on the relative time of occurrence of the events to obtain an output indicative of a further medical event. According to certain embodiments, there is provided a non-transitory computer readable medium storing the computer program.

Embodiments will be described in more detail with reference to the accompanying Figures.

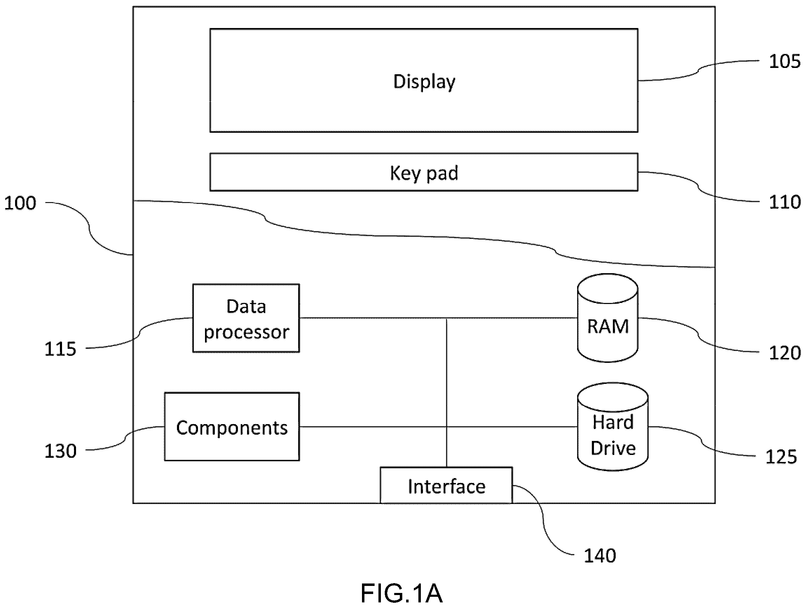

Reference is made to Figure 1A, which illustrates a data processing apparatus 100, which takes the form of a computing device. The processing performed to implement the method for determining the occurrence of an event that has taken place during a clinical procedure is performed by the data processing apparatus 100. The apparatus 100 may be a mobile user equipment (UE), a personal computer (PC), a terminal or workstation, a server, or some other form of device.

The apparatus 100 comprises an interface 140 over which it sends and receive signals. The interface 140 may be a wired or wireless interface. For instance, the interface 140 may comprise a wired interface for connection to a wired network (e.g. a local area network and/or the internet). Alternatively or in addition, the interface 140 may comprise transceiver apparatus configured to send and receive communications over a radio interface. The transceiver apparatus may be provided, for example, by means of a radio part and associated antenna arrangement. The antenna arrangement may be arranged internally or externally to the apparatus 100.

The apparatus 100 is provided with at least one data processing entity 115, at least one random access memory 120, at least one read only memory 125, and other possible components 130 for use in software and hardware aided execution of tasks it is designed to perform, including control of, access to, and communications with access systems and other communication devices. The at least one random access memory 120 and the hard drive 125 are in communication with the data processing entity 115, which may be a data processor. The data processing, storage and other relevant control apparatus can be provided on an appropriate circuit board and/or in chipsets. A user controls the operation of the apparatus 100 by means of a suitable user interface such as key pad 110, or by voice commands. A display 105 is included on the apparatus 100 for displaying visual content to a user. The apparatus 100 may also comprise a speaker for providing audio content.

The memory of the apparatus 100 (i.e. the random access memory 120 and the hard drive 125) may be configured to store computer readable instructions for execution by the data processor 115 to perform the data processing functions described herein as being performed by the apparatus 100. Alternatively, the components 130 may comprise hardware components, such as a field programmable gate array (FPGA) or application specific integrated circuit (ASIC), for performing the operations described herein as being performed by the apparatus 100. In some embodiments, the operations described herein as being performed by the apparatus 100 may be performed by a combination of the hardware components or by a processor executing computer readable instructions.

Although the apparatus 100 is shown as a single unified device 100, in other embodiments, the apparatus 100 may comprise a plurality of interconnected devices.

The apparatus 100 may receive data according to a plurality of different modalities. Each of these modalities may be referred to as a different data channel. Data of each modality may be received from a different data collection device.

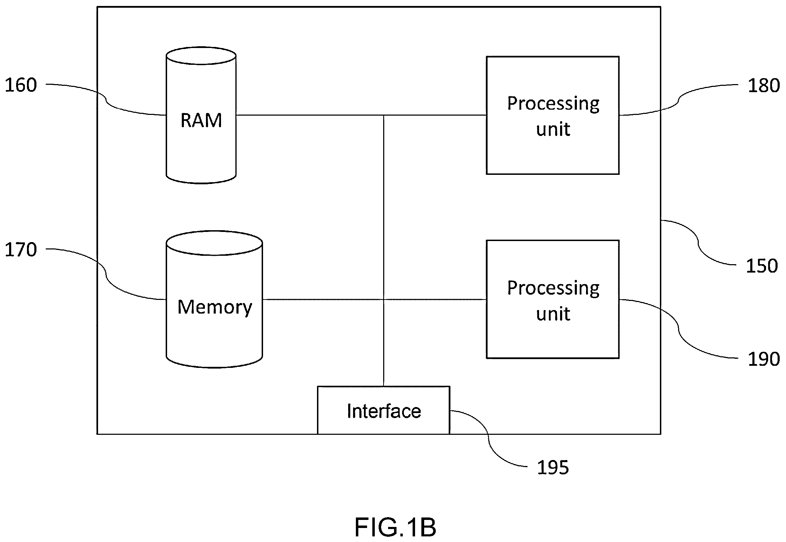

Reference is made to Figure 1B, which illustrates an example computer apparatus 150 that may be used for performing the training of machine learning models, which may be used to perform some of the processing described herein. The apparatus 150 is shown as a single enclosed apparatus. However, in some embodiments, the apparatus 150 is a distributed system, with multiple data processing apparatuses operating in communication with one other. The apparatus 150 may comprise a server, back-end system, or the like.

The apparatus 150 comprises at least one random access memory 160, at least one hard drive 170, at least one data processing unit 180, 190 and an input/output interface 195. The memories 160, 170, store data for inputting to the one or more models and for storing results of the processing performed during execution of the one or more models. The memories 160, 170 store the training data, which is applied to train the machine learning models. The memories 160, 170 additionally store computer executable code which, when executed by at least one data processing unit 180, 190, provide the one or more machine learning models. At least one of the data processing units 180, 190 performs one or more of: the processing associated with the one or more models, the training of the models, and any necessary pre-processing of data for use by the models. Via the interface 195, the apparatus 150 receives the data items for constructing the training data sets and/or the data items for constructing the operating data sets. The apparatus 150 additionally sends via the interface 195, the results produced by running the models on input data.

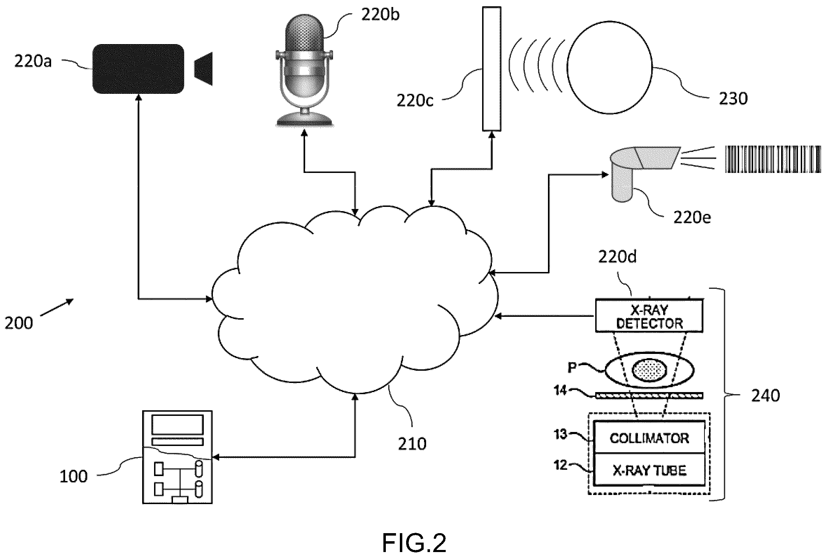

Reference is made to Figure 2, which illustrates an example of a system 200 comprising the apparatus 100, which receives the data according to the different modalities and processes this data, and a plurality of example data collecting devices 220a-e. The data collecting devices 220a-e may communicate with the apparatus 100 over a network 210.

The example data collecting devices 220a-e may include a camera 220a for obtaining a video of a clinical procedure. In the present embodiment, the video indicates a still image (camera image) and a moving image (video). The camera 220a could be a camera that is installed on a ceiling, etc., of a clinic and recoding a video of the inside of the clinic, in which case the video obtained by the camera 220a may show a patient, medical equipment, and/or medical staff members during the clinical procedure. More particularly, the camera 220a could be a camera for showing, for example, an area near a hand of a medical staff member when the medical staff member (operator) operates a certain medical device. For example, the medical equipment includes a contrast-agent injecting device, an anaesthetic machine, a guidewire, an indwelling device (MitraClip, a medical device (stent, balloon, or the like) used in a percutaneous coronary intervention (PCI), a pressure sensor used for a fractional flow reserve (FFR) assessment, a prosthetic valve used in a transcatheter aortic valve implantation (TAVI), etc). The camera 220a could also be a camera for showing a medical staff member (doctor, nurse, etc.) or a patient who enters the operating room. Moreover, the camera 220a could be part of an endoscope used for obtaining video data of the inside of a patient. The video data obtained by the camera 220a is transmitted to the apparatus 100. The example data collecting devices 220a-e may include a microphone 220b for obtaining audio data collecting during the clinical procedure. The example data collecting devices 220a-e may include an RF detector 220c, which detects the presence of an RF tag 230 in close proximity to the RF detector 220c. Such an RF tag 230 may be attached to a piece of medical equipment and may be scanned against the detector 220c before use of the medical equipment by a medical staff member. The data collecting devices 220a-e may include an x-ray detector 220d for obtaining X-ray imaging data of the patient p during a clinical procedure. The x-ray detector 220d may be part of X-ray imaging equipment 240, which also includes an x-ray tube 12 for generating the x-rays and a collimator 13 for restricting the field of view of the X-ray tube 12. The X-ray imagining apparatus 240 may further include a filter 14 for filtering the X-ray beam output by the X-ray tube 12.

The data collecting devices 220a-e may include a barcode scanner 220e, which is used for detecting and reading barcodes. Such a barcode may be attached to a piece of medical equipment and may be scanned by the barcode scanner 220e before use of the medical equipment by a medical staff member.

Therefore, each of the example data collecting devices 220a-e collects data belonging to a different data modality and provides this data to the apparatus 100. The apparatus 100 thus receives the data belonging to a plurality of channels. Each of the data collecting devices 220a-e collects the data in a synchronous manner. In other words, the data collecting devices 220a-e collect the data with reference to a common timeline for the clinical procedure, and each item of data collected (e.g. a frame of a video) is labelled with time information indicating its position in the timeline. The data collecting devices 220a-e provide the time information along with the data belonging to the plurality of channels to the apparatus 100. Each of the devices 220a-e may operate according to a common system clock, which is used to provide time information associated with the data it records throughout the procedure.

The example data collecting devices 220a-e are given as examples only, but there may be other types of data collecting device that are part of system 200 and which provide data of a given data modality to the apparatus 100. For example, alternatively to or in addition to fluoroscopy, the other types of data collecting device may collect other types of imaging data - such as positron emission tomography (PET) data or magnetic resonance imaging (MRI) data. At least one of the data channels includes imaging data - which may take the form of video data collected by the camera 220a or medical imaging data obtained using an imaging technique, such as fluoroscopy, DA acquisition ,CT data acquisition ,MR data acquisition. Moreover, for example, the other types of data collecting device may collect ultrasonic image data by ultrasonic scanning. Note that the ultrasonic scanning can be considered as an example imaging technique. In this case, the ultrasonic image data can be considered as an example of medical imaging data. Moreover, the other types of data collecting device may collect vital data (for example, an electrocardiogram waveform, a heart rate, a respiratory waveform, a respiratory rate, a blood pressure, a body temperature, a percutaneous oxygen saturation (SpO2), etc).

The apparatus 100 provides a plurality of modules for processing data belonging to the different channels received at the apparatus 100 and for deriving from the data belonging to the different channels, labels indicative of events that have occurred in the data belonging to the different channels. Each of these modules is referred to herein as a 'watcher'. Each such watcher may be a software module running on the processor 115 of the apparatus 100 or could be implemented in hardware. The watchers identify basic events, such as the type of anatomy in a medical image, the presence of people in a room in which the procedure is taking place, or a given type of interaction with a piece of medical equipment. The events identified by the watchers are referred to herein as sub-events, so as to distinguish them from the higher-level events identified on the basis of the labels output by the watchers. A model, which may be a machine learning model/s or a state machine, classifies at a higher level, using only labels and their position in the timeline to understand what is happening in the room.

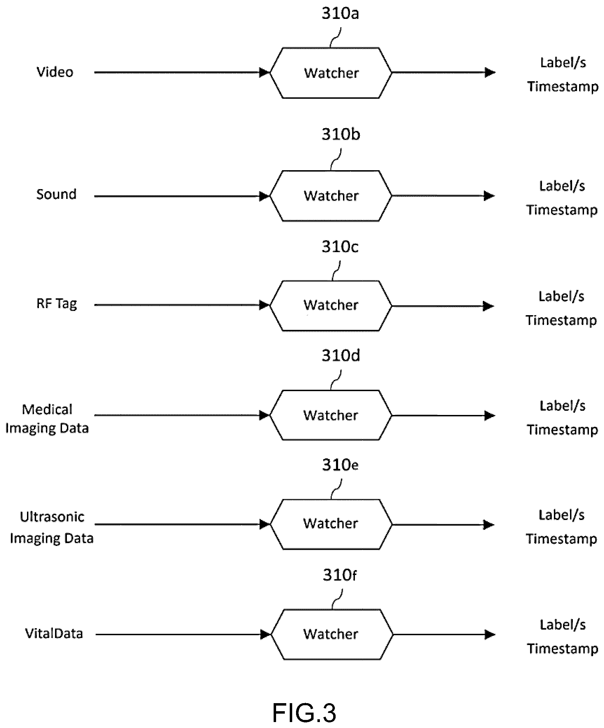

Reference is made to Figure 3, which illustrates a plurality of watchers 310a-f, each of which is associated with a different data channel. Each of the watchers 310a-f receives data belonging to the particular data channel with which it is associated and may identify a sub-event indicated by the received data. Upon identifying a sub-event, each watcher 310a-f outputs a label indicative of the sub-event. Each such label takes the form of one or more numerical values or a string that identifies the type of sub-event and that are suitable for input into a model (e.g. a machine learning model or a classical state machine) to identify an event indicated by a number of the sub-events. Each watcher 310a-f, in addition to outputting the label/s indicative of a detected sub-event, also outputs a timestamp indicating the time at which the sub-event took place. The apparatus 100 may use the timestamps to determine a time for the event identified based on the labelled.

Note that, in the present embodiment, the watcher 310a-f may output a label indicative of a sub-event that is detected based on a rule (data table, etc), instead of a model.

The watcher 310a receives video data. The watcher 310a may identify a sub-event in the video data. The sub-event may, for example, be a specific action of a medical staff member (e.g. preparing an injection), or could be a particular structure recognised on an endoscopy video. The watcher 310a outputs on the basis of the identified sub-event labelled in the video data, one or more numerical values or a string representing a label of the sub-event.

The watcher 310b receives audio data. The watcher 310b may identify a sub-event in the audio data. The sub-event may comprise a particular spoken word or phrase or a spoken phrase having a particular semantic meaning (for example, phrases such as "A catheter is inserted", "Injection of a contrast agent starts", and "An anaesthetic agent is injected"). The watcher 310b may employ automatic speech recognition to identify words belonging to the audio data. The watcher 310b may additional apply natural language understanding to determine a semantic meaning of the identified words. The watcher 310b may match the identified words and/or determined semantic meaning against a particular set of identified words and/or determined semantic meanings in order to identify a particular label.

The watcher 310c receive as an input, data indicating whether or not radio frequency (RF) waves have been detected as a result of the presence an RF tag in close proximity to a detection apparatus. The watcher 310c outputs a label indicating that the RF tag was detected and a timestamp indicating the time of detection.

The watcher 310d receives as an input, medical imaging data. The medical imaging data may comprise fluoroscopy data. The watcher 310d may identify a sub-event in the medical image data. For example, the medical imaging data could be used to identify a portion to be treated (heart, head portion, thoracic portion, etc). Moreover, for example, the medical imaging data could be used to measure the position of a guidewire or indwelling device inserted into a patient. In this case, the sub-event may, for example, be the arrival of the guidewire or indwelling device at a given position within the patient. The watcher 310d outputs on the basis of the identified sub-event labelled in the medical imaging data, a label representing the sub-event. The watcher 310d additionally outputs a timestamp indicating the time at which the sub-event took place.

Note that the watcher 310d may display, on the display 105, medical image data (CT data, MR data) captured before the operation as a reference image in addition to the outputting of the label of the sub-event as described above.

Moreover, the watcher 310d may receive state information indicative of a state of a medical device. If the medical device is the X-ray imaging equipment 240, for example, the state information of the medical device could be an angle of a C-arm, a position of the X-ray detector 220d, positional information of an X-ray exposure field (identified from a mechanical position of an aperture blade, for example), positional information of a table, etc. The watcher 310d may identify a sub-event in the medical imaging data from the state information of the medical device. The watcher 310d may also identify a sub-event indicating that an image of a particular portion of a patient is being captured based on the collected medical imaging data.

The watcher 310e receives ultrasonic image data as input. The watcher 310e may identify a sub-event in the ultrasonic image data. For example, an ultrasonic image could be used to measure a position of a medical device, such as a catheter, etc., inserted into a patient. In this case, the sub-event may be, for example, arrival of the catheter, etc., at a given position within the patient. The watcher 310e outputs on the basis of the identified sub-event labelled in the ultrasonic image data, a label representing the sub-event. The watcher 310e additionally outputs a timestamp indicating the time at which the sub-event took place.

Note that the watcher 310e may display, on the display 105, ultrasonic image data collected before the operation as a reference image in addition to the outputting of the label representing the sub-event.

The watcher 310f receives vital data as input. The vital data may include, for example, an electrocardiogram waveform, a heart rate, a respiratory waveform, a respiratory rate, a blood pressure, a body temperature, an SpO2, etc. The watcher 310f may identify a sub-event in the vital data. The watcher 310f outputs on the basis of the identified sub-event labelled in the vital data, one or more numeric values or a character string representing a label of the sub-event.

Note that the watcher 310f may display, on the display 105, the vital data in addition to the outputting of the label of the sub-event as described above.

Moreover, the watcher 310 may receive state information indicative of a state of a medical device. If the medical device is the X-ray imaging equipment 240, for example, the state information of the medical device could be an angle of a C-arm, a position of the X-ray detector 220d, positional information of an X-ray exposure field (identified from a mechanical position of an aperture blade, for example), positional information of a table, etc. Moreover, if the medical device is a contrast agent injecting device (injector) for automatically injecting a contrast agent, for example, the state information could be information indicative of a timing to start injection of the contrast agent (identified by reception of an operation (button operation) to instruct a start of injection, for example), etc. Furthermore, if the medical device is an anaesthetic machine, for example, the state information could be information indicative of a timing to start administration of an anaesthetic agent (identified by reception of an operation (button operation) to instruct a start of administration, for example), etc. The watcher 310 may identify a sub-event from the above-mentioned information.

The watcher 310 may identify a sub-event indicative of a type of X-ray imaging (fluoroscopy imaging, contrast radiography, CAG imaging, etc.) from various information received.

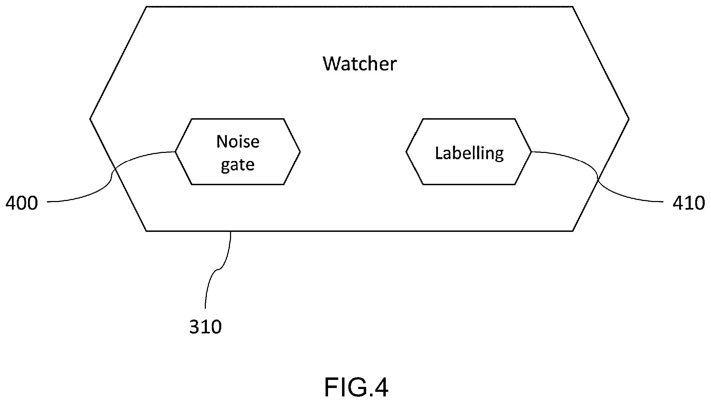

Reference is made to Figure 4, which illustrates examples of components that may belong to a watcher 310. The watcher 310 may be any of the watchers 310a-f illustrated in Figure 3.

The watcher 310 comprises a noise gate module 400. The noise gate 400 monitors a stream of data received on the data channel with which the watcher 310 is associated. The noise gate 400 identifies when a change takes place in the data on the channel that may be indicative of a sub-event. The watcher 310 further comprises a labelling module 410. The labelling module 410 is applied when the noise gate 400 identifies activity in the data on the channel. The labelling module 410 receives the data and - when the noise gate 400 is triggered (i.e. identifies activity in the data) - identifies and performs a classification of this data to determine whether a sub-event belonging to one of a predetermined set of sub-events defined for the watcher 310 has taken place. If the labelling module 410 determines that one of these sub-events has taken place, the labelling module 410 outputs the label indicating that sub-event.

For example, the watcher 310 may be the watcher 310b that is used to receive audio data. In this case, the noise gate 400 belonging to the watcher 310 receives a stream of audio data and may identify when there is a change in volume the audio data, which may indicate, for example, speech. Upon identifying a point in the audio data at which speech occurs, the noise gate 400 provides an indication of this to the labelling module 410. The labelling module 410 may then apply a speech recognition model to identify the words spoken at the point in the audio data. The labelling module 410 may further apply natural language understanding to the words identified in the audio data to determine a semantic meaning and to identify a label based on the identified semantic meaning. As a further example, the noise gate 400 may detect movement in a stream of video data, and then trigger the labelling module 410 to identify a sub-event taking place in the video, e.g. the presence of one or more people.

The labels of sub-events derived from a plurality of watchers and the relative order and timing of the sub-events are used to identify events indicated by the plurality of sub-events. For example, the sub-event labels may be input into a machine learning model (such as a recurrent neural network) in the order in which they are generated in order to derive a suitable event label.

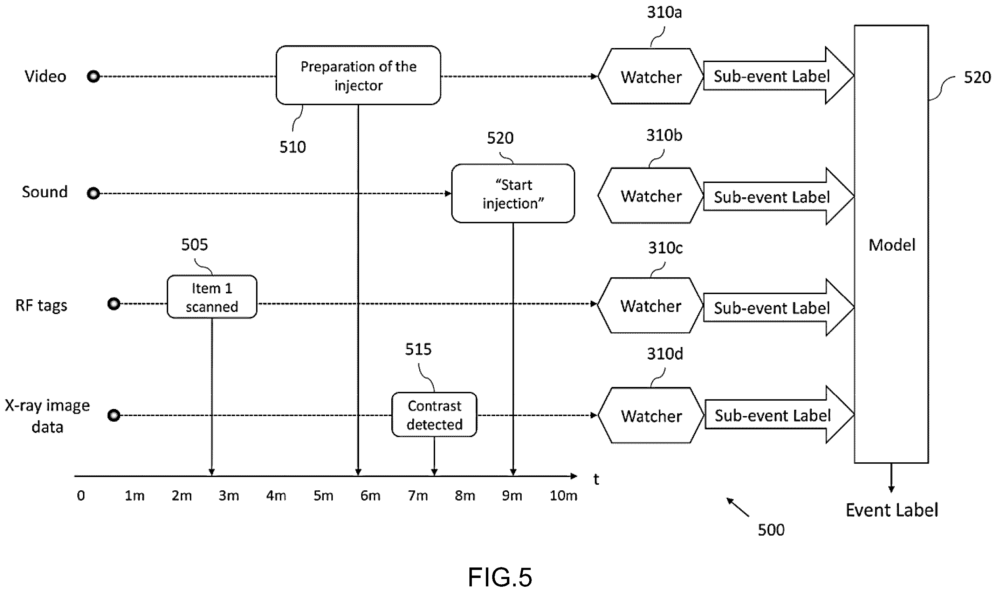

Reference is made to Figure 5, which illustrates an example of a timeline covering a time period during which a plurality of sub-events are detected in different data channels.

At 505, the presence of an RF tag is detected. This RF tag is associated with a particular item (item 1), and may be attached to an outer covering or casing of that item. In embodiments, when the item is retrieved for use by a medical staff member, the tag associated with the item is scanned to indicate that the item has been retrieved. In this case, the detection apparatus 220c sends a signal to the device 100 indicating that the RF tag associated with item 1 has been scanned. The device 100 identifies the scanning of item 1 as being a sub-event and derives a label identifying this sub-event along with a timestamp for the sub-event.

Note that at 505, it may be detected that a code symbol, such as one- or two-dimensional code, has been scanned. In this case, the code symbol is associated with a particular item (item 1), and may be attached to an outer covering or casing of that item. For example, when the item is retrieved for use by a medical staff member, the code symbol associated with the item is scanned by a reading device to indicate that the item has been retrieved. In this case, the reading device may send a signal to the device 100 indicating that the code symbol associated with item 1 has been scanned. The device 100 may identify the scanning of item 1 as being a sub-event and derive a label identifying this sub-event along with a timestamp for the sub-event.

At 510, on the video data channel, a video of a nurse preparing an injection for administering to a patient is recorded. The camera 220a supplies a stream of video footage to the device 100, which analyses (using the watcher 310a) the stream of video footage to identify in the stream of video footage, the point in the video footage at which a nurse prepares the injection. The device may analyse the video footage by applying one or more convolutional neural networks to the video, in order to identify part of the video that represents preparation of an injection by a person. Upon identifying this part of the video, the device 100 derives a label representing the sub-event (i.e. preparation of the injection) and a timestamp representing the time at which the sub-event took place.

At 515, on the fluoroscopy data channel x-ray imaging data is recorded. Fluoroscopy is an example of an X-ray imagining technique for obtaining a stream of X-ray images, but the X-ray imagining equipment 240 could operate otherwise to provide one or more X-ray images. The X-ray imaging equipment 240 supplies a stream of x-ray imaging data to the apparatus 100, which analyses (using the watcher 310d) the stream of imaging data to identify in the imaging data, the point at which contrast is detected. Upon identifying this part of the imaging data, the apparatus 100 derives a label representing the sub-event and a timestamp representing the time at which the sub-event took place.

The X-ray imaging data, herein, includes a captured image that is an X-ray image with a high dose and a fluoroscopic image that is an X-ray image with a low dose.

At 520, on the audio data channel, audio is recorded in which the words "start injection" are present. The microphone 220b supplies a stream of audio data to the device 100, which analyses (using the watcher 310b) the stream of audio data to identify in the stream of audio data, a point at which words are spoken. The spoken words are analysed by applying a speech recognition algorithm to identify the words. The words may be further analysed to determine a semantic meaning. Upon determining that the words indicate an instruction to start or perform an injection, the device 100 derives a label indicative of a sub-event at which an instruction to start or perform an injection were spoken. The device 100 further derives a timestamp indicative of the time at which the sub-event took place.

In this example, the device 100, therefore, obtains various labels indicative of different sub-events that have taken place on different data channels. Each of these labels is providing in the form of one or more numerical values or a text string that is suitable for input into a model 520. The device supplies the labels as inputs to the model 520 in order to obtain a label for an event that is indicated by the labels for the sub-events. For example, given the three sub-events 505, 510, 515, 520 shown in Figure 5 and the corresponding labels derived by the watchers 310a-c, the model 520 may output a set of values indicating an event that is the preparation and administration of an injection.

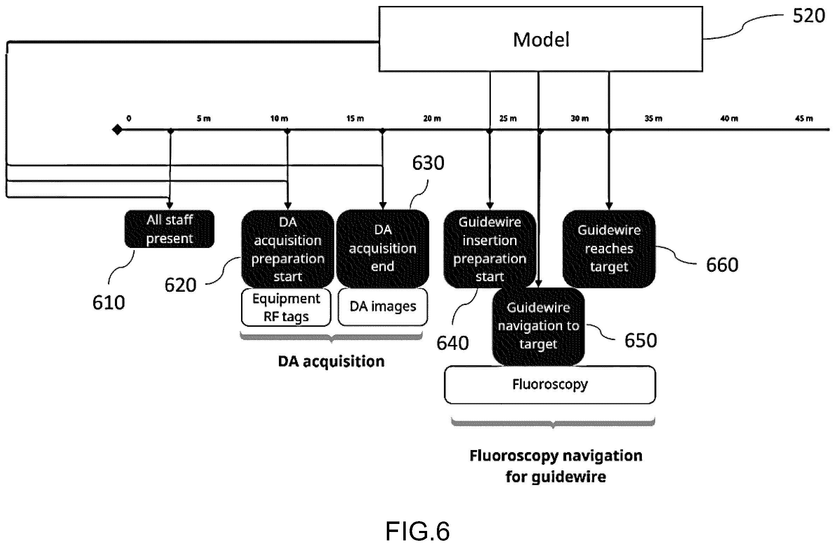

To ensure that the relative timing of the events is taken into account in deriving the event label, the watchers process each of the data streams (i.e. the data belonging to different modalities) in real time and generate sub-event labels when sub-events occur. The device 100 may be configured to input each of the sub-event labels upon generation of each label. In this way, the time of input of the sub-event label into the model 520 corresponds to the time at which the sub-event occurs in the common timeline. In the example of Figure 5, when the first sub-event 505 occurs at approximately 2:45 in the timeline, the watcher 310c derives the appropriate sub-event label and the device 100 supplies this as an input this into the model 520, which causes the state of the model to be updated. Subsequently, when the second sub-event 510 occurs at approximately 5:55 in the timeline, the watcher 310a derives the appropriate sub-event label and the device 100 supplies this as an input this into the model 520, which causes the state of the model 520 to again be updated. Subsequently, when the third sub-event 515 occurs at approximately 7:20 in the timeline, the watcher 310d derives the appropriate sub-event label and the device 100 supplies this as an input into the model 520, which causes the state of the model 520 to again be updated. Subsequently, when the fourth sub-event 520 occurs at approximately 9:05 in the timeline, the watcher 310b derives the appropriate sub-event label and the device 100 supplies this as an input into the model 520, which causes the state of the model 520 to again be updated. Following the multiple updates to the state of the model 520 resulting from the sub-event label inputs, the output of the model 520 represents an event label for an event indicated by the plurality of the sub-events. In this example, the indicated event may be the start of digital angiography preparation 620 shown in Figure 6 as an example.

Taking into account the relative timing of sub-events may enable sub-events that took place a long time ago, and therefore may be unrelated to more recent sub-events to be discounted in the identification of an event label.

The model 520 may take the form of a recurrent neural network (RNN), which is configured to store state in relation to past sub-events. Such a recurrent neural network may be configured to update its state based on the relative timing of sub-events. For example, the network may be configured to 'forget' sub-events that occurred a significant amount of time earlier in the common timeline.

The model 520 may be a classical state machine, which is configured to store state in relation to past sub-events and update that state in response to further sub-event labels to derive one or more event labels.

In some embodiments, the relative timing of sub-events may be accounted for by processing the timestamps generated for the sub-events in order to derive the event labels. These timestamps may be provided as inputs to a model 520, in addition to the sub-event labels.

The different events for which labels are output by the model 520 may include clinical events, stages of a procedure, and/or actions by staff. These labels may be used to index and summarise procedures. Reference is made to Figure 6, which illustrates an example of multiple different labels of events that may be output over the course of a procedure. Figure 6 shows how these labels are output at different points in time during the procedure. Each of these labels is output on the basis of one or more different sub-events.

As shown in Figure 6, a first label 610 for an event that is output by the model 520 is that all staff for a procedure are present. The model 520 may output this first label 610 in response to receiving as an input, labels indicating the entry of staff members to a room in which the procedure takes place. Such labels of sub-events may output by watcher 310 on the basis of video footage. Additionally, the model 520 may output the first label 610 in response to receiving as an input, labels indicating sub-events detected in the audio data received at the device 100. Such sub-events detected in the audio data may include detection of speech by different staff members, names of certain staff members being spoken, or phrases having a certain semantic meaning (e.g. 'everyone present') being spoken.

As shown in Figure 6, a second label 620 for an event that is output by the model 520 is the preparation for the obtaining of digital angiography (DA) images. This label 620 may be derived by the apparatus 100 on the basis of: a label indicating a sub-event that an RF tag attached to a piece of equipment for performing the imaging was scanned against a detector, a label indicating a sub-event that video data showing the preparation of an injector, and a label indicating a sub-event that audio relating to the start of injection was recorded by the microphone 220b.

As shown in Figure 6, a third label 630 for an event that is output by the model 520 is the end of DA acquisition. This label 630 may be output by the model 520 on the basis of a label representing a sub-event that the end of a series of DA images has been reached. This sub-event may be detected on the basis of a stream of DA imaging data received at the device from an apparatus for performing DA imaging(Digital Angiography imaging). The label 630 may additionally be output on the basis of a label indicating a sub-event detected in audio data, e.g. a spoken phrase indicative of the end of the DA imaging procedure.

As a further example, a label indicating the acquisition of a DA image may be output by the model 520 on the basis of a first label representing a sub-event at which contrast diffusion in the vasculature in clinical images is detected, a second label representing a sub-event at which a clinician asks for contrast agent to be injected, and a third label representing a sub-event at which a barcode of a fresh vial of contrast agent is scanned.

It would be appreciated by the skilled person that DA imaging is an example imaging type for which event labels may be output at 620 and 630, but that other imaging types or ultrasonic scanning may be used to output event labels at 620 and 630.

As shown in Figure 6, a fourth label 640 for an event that is output by the model 520 is the insertion of a guidewire into a patient. This label 640 may be output by the model 520 on the basis of a label representing a sub-event that fluoroscopy imaging has begun, where that label is derived by the device 100 on the basis of fluoroscopy imaging data received at the apparatus 100 from the device 220d. The label 640 may be output in dependence upon a label representing one or more sub-events detected in the audio data relating to the guidewire.

A fifth label 650 for an event that is output by the model 520 is the navigation of the guidewire to the target. This fifth label 650 may be output by the model 520 in response to receipt at the model 520 of a label representing a sub-event that the guidewire is in motion, where that label is derived from fluoroscopy imaging in which the guidewire is shown. Additional labels of sub-events determined from additional data channels may be also be supplied as inputs to the model 520 in order to derive the label 650.

A sixth label 660 for an event that is output by the model 520 is the guidewire reaching the target. This sixth label 660 may be output by the model 520 in response to receipt at the model 520 of a label representing a sub-event that the guidewire has stopped moving, where that label is derived from fluoroscopy imaging in which the guidewire is shown. Additional labels of sub-events determined from additional data channels may be also be supplied as inputs to the model 520 in order to derive the label 660.

As shown in the example of Figure 6, each of the event labels is allocated a location on the common timeline. The time for each event label may be derived by the apparatus 100 from the timestamps of the sub-event labels used to derive the respective event label. For example, for a particular event label, that event label may be allocated a time that is the latest (or shortly after the latest) of the timestamps of the sub-events used to derive the event label. In the example of Figures 5 and 6, the event label 620 is allocated a time that shortly follows the timestamp of the sub-event 520.

In the examples discussed, the model 520 may comprise one or more neural networks that receive the sub-event labels as an input and provide a corresponding event label as an output. Furthermore, one or more of the watchers 310 may each comprise one or more neural networks, where each of the neural networks receives the sub-event labels as an input and provides a corresponding event label as an output.

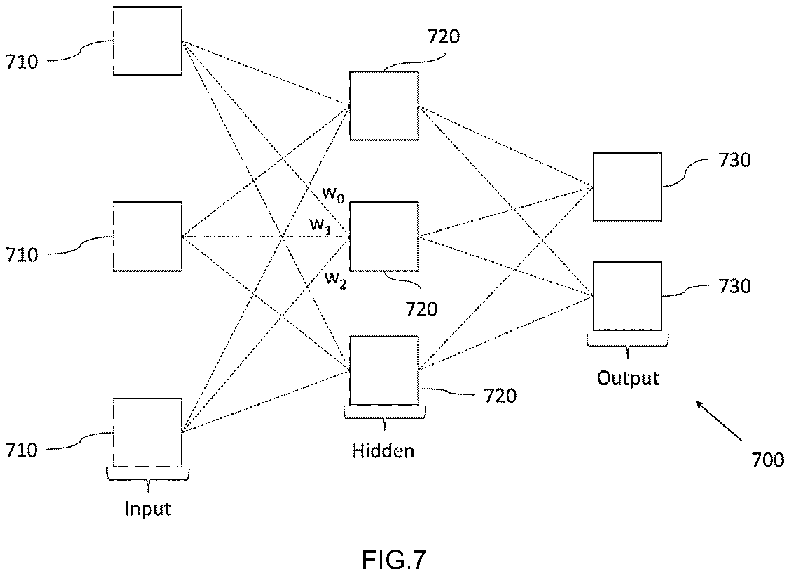

Figure 7 as a schematic illustration of a neural network 700. The neural network 700 comprises input nodes 710, hidden nodes 720 and output nodes 730. In practice, there are likely to be many more nodes in the network 700 than those shown, and more hidden layers than the one shown. Each input node 710 receives a single value of the input data and produces at its output, an activation or node value, which is generated by supplying the input value to an activation function (e.g. a sigmoid). Each of the input nodes 710 is connected to each of the hidden nodes 720. A matrix of weights defines the connectivity between the input nodes 710 and the hidden nodes 720. A vector of the node values output from the input nodes 710 is scaled by a vector of respective weights at the input of each of the hidden nodes 720, each weight defining the connectivity of one of the input nodes 710 with a connected one of the hidden nodes 720. The weights applied at the inputs of one of the hidden nodes 720 are shown in Figure 7 as w0....w3. At each hidden node 720, the input value at that node is given by the dot product of its associated weights vector and the output values of the input nodes 710. The activation function is then applied to the input values at the hidden nodes 720 to provide the output values of those nodes 720. The output vector of the hidden nodes 720 is supplied to each of the nodes 730 in the next layer of the network 700 and used in a similar manner to generate the output values for that next layer.

The network 700 may be trained through supervised or unsupervised learning. In one embodiment, the network 700 is trained through supervised leaning by determining at least one set of output values based on at least one set of input values included in the training data. The output values are compared to known labels in the training data and an error or loss is calculated (i.e. based on a difference between the output values and the labels). The error or loss is then back-propagated through the network 700 to update the weights, such that the network 700 is trained to better approximate the labels from the input values. In the next cycle, the revised weights are used with further training data to further update the weights to more closely reproduce the labels of the further training data based on the input values of the further training data. In this way, the network 700 can be trained to perform a specific task.

When performing video or image classification, a convolutional neural network may be used. Convolutional neural networks are neural networks that make use of a convolution calculation in at least one of their layers. Convolutional neural networks are particularly well adapted to image analysis and processing as they are shift invariant. To perform recognition of features in a video or series of medical images, 2D convolutional neural networks (CNN) may be applied to identify features in individual frames in a video or series of medical images. Alternatively, to perform recognition of features in a video, 3D convolutional neural networks (CNN) may be applied to identify features within a video, including identifying temporal relationships between frames.

Alternatively to or in addition to the use of CNNs, the video or image classification may be performed using traditional image analysis algorithms.

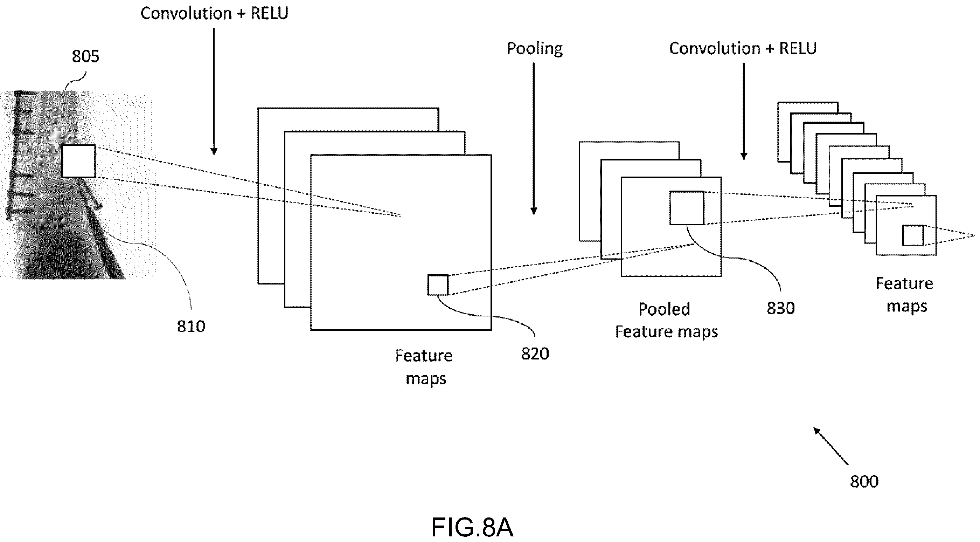

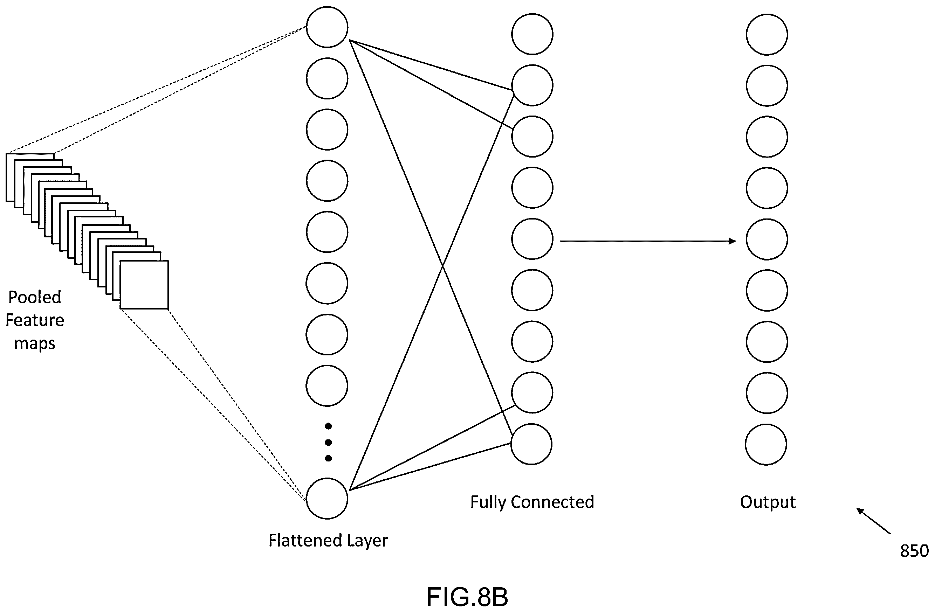

Reference is made to Figures 8A and 8B, which illustrate an example of the operation of a convolutional neural network, which can be used to identify certain features within frames of a video and perform classification of those features. In the example shown, the input image is an X-ray image 805 showing a plurality of implants inserted into the patient. The convolutional neural network may be used to identify when each of the implants is positioned at its final location during surgery.

A kernel 810 is applied to determine a convolution of the input image 805 with the kernel 810. The output of this convolution is subject to an activation function to add nonlinearly. The activation function used in Figure 8A is a rectified linear activation unit (RELU), which, if the input is positive, outputs the input, and, if the input is not positive, outputs zero. A plurality of feature maps are generated from the input image by performing convolutions between the input image and different kernels, where each kernel represents a different basic feature, e.g. a vertical line or horizontal line.

Each of the feature maps produced by the convolution and activation function is then subject to a pooling process, which is performed to reduce the spatial size of the convolved feature. The pooling process involves translating a kernel across the feature map to sample groups of pixels and returning the maximum or average value from each of the sampled groups of pixels in the feature map. The resulting pooled feature maps are each subject to a further convolution process (with the RELU function applied) using the different kernels to generate a further set of feature maps from which pooling is again performed.

As shown in Figure 8B, the pooled feature maps resulting from multiple stages of convolution and pooling are flattened to produce a one dimensional array (shown as Flattened Layer), which is provided as a set of input values to a feed forward neural network. The resulting output values represent the state of the implants in the X-ray image. The apparatus 100 may process the output values to infer whether or not the implants are at their final location in the patient.

The convolutional neural network may be trained by comparing output values for different images to labels of those images and adjusting the weights of the feed forward portion of the convolutional neural network.

As noted, the model 520 receives as inputs different labels of sub-events in a sequence in order to derive an output representing an event. To ensure that the output of the model 520 is dependent upon the relative timing of the sub-events, in some embodiments the model 520 may comprise at least one recurrent neural network for processing the inputs.

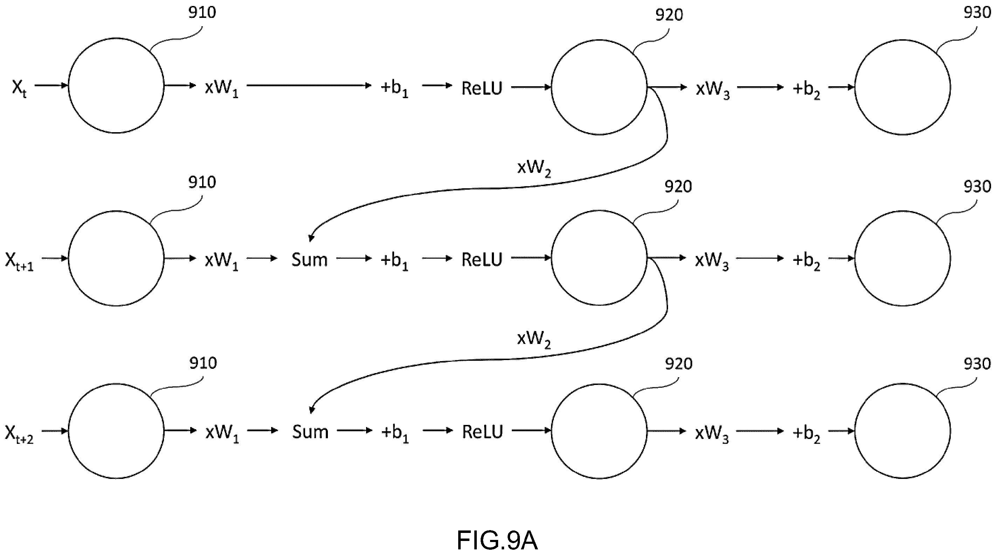

Reference is made to Figure 9A, which illustrates a simple example of a recurrent neural network (RNN), having an input node 910, a hidden layer node 920, and an output node 930. A plurality of sets of input data are provided as inputs to the RNN at different points in time. A first set of input data Xt is provided as an input at time t for a first iteration of the RNN, a second set of input data Xt+1 is provided at a subsequent time t+1 for a second iteration of the RNN, a third set of input data Xt+2 is provided at a further subsequent time t+2 for a third iteration of the RNN. In this simple example, each set of input data comprises only a single value.

To calculate the activation value at the hidden layer node 920 during the first iteration of the RNN, the input value Xt is multiplied by the weight W1. The bias b1 is added to the result of this multiplication, and the result of the addition is provided an activation function (ReLU). The output of the activation function (e.g. a rectified linear unit (ReLU) function) provides the activation for the hidden layer node 920. The activation of the hidden layer node 920 may be subject to further processing (i.e. multiplication by weight W3 and the addition of the bias b2) to generate the activation of output node 930. However, the activation of the output node 930 may be either not calculated or ignored until all of the sets of input values have been processed.

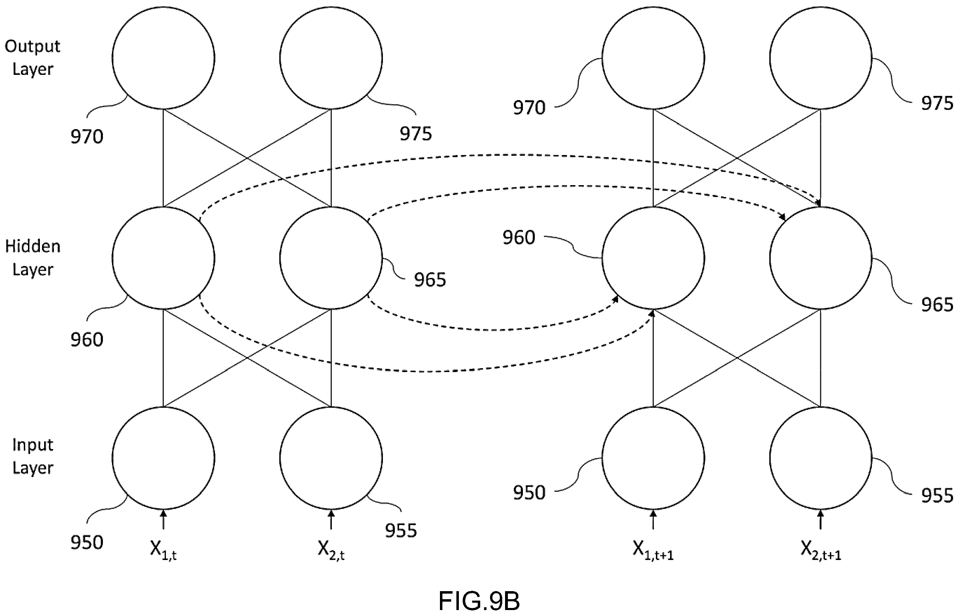

The activation for the node 920 determined when performing the first iteration constitutes hidden state, which is used when processing the next input value Xt+1 as part of the second iteration of the RNN. When the next input value Xt+1 is processed by the node 910, it is also multiplied by the weight W1. The result of this multiplication is added (shown as "Sum" in Figure 9) to the hidden state of node 920 determined in the first iteration. The result of this sum is then added to the bias b1, and then supplied to the activation function to determine the activation of the hidden layer node 920 for the second iteration. The activation for the hidden layer node 920 calculated for the second iteration is then used when processing the third input value Xt+2 to calculate the activation of the hidden layer node 920 for the third iteration.

The processing of multiple sets of input values may continue in the manner described with the hidden state for each proceeding iteration of the RNN being used for the current iteration until the final set of inputs is processed in a final iteration. The activation of the output node 930 for the final iteration provides the output of the RNN.

Figure 9A represents a simplified example of an RNN having only three nodes 910, 920, 930. Figure 9B represents a further example in which the RNN comprise two nodes in each layer. This further example RNN comprises two nodes 950, 955 in the input layer, two nodes 960, 965 in the hidden layer, and two nodes 970, 975 in the output layer. In this case, the RNN comprises multiple states that are used when processing a next set of input values. A first set of input values X1,t, X2,t are provided as inputs to nodes 950, 955 of the input layer for processing in a first iteration. Each of the activations of the hidden layer nodes 960, 965 calculated during this iteration are then used to calculate each of the activations of the hidden layer nodes 960, 965 during a second iteration in which a second set of input values X1,t+1, X2,t+1 are processed.

As discussed above, ones of the watchers 310 may be implemented using machine learning models that are used to provide labels of sub-events. To provide each of these models, training processes are performed by apparatus 150 to train the models using sets of training data.

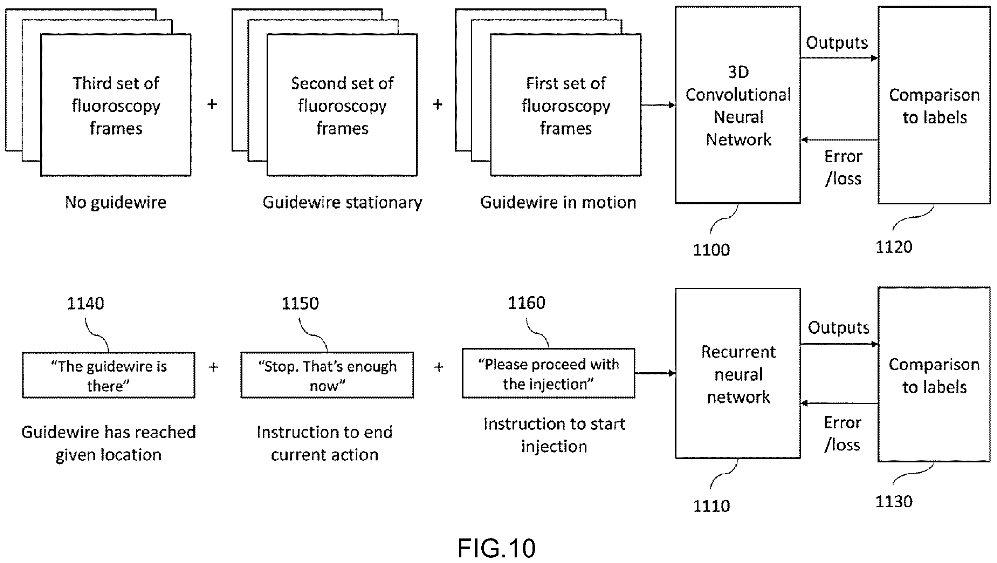

Reference is made to Figure 10, which illustrates how two example machine learning models 1100, 1110 may be trained. The machine learning model 1100 is a 3D convolutional neural network for identifying sub-events detected in a series of x-ray images/frames. To train the model 1100, a plurality of sets of x-ray frames are provided to the apparatus 150, where each of those sets have been labelled by a human user. Figure 10 shows a first set of frames labelled as showing a case in which a guidewire is in motion, a second set of frames labelled as showing a case in which a guidewire is stationary, and a third set of frames labelled as showing images in which no guidewire is shown. Each of those set of frames are input by the apparatus 150 to the 3D convolutional neural network 1100 to derive a set of outputs. For each set of frames, the outputs are compared to the corresponding label a comparison stage 1120 to determine an error/loss, which is then used to update the parameters of the model 1100.

The machine learning model 1110 is a recurrent neural network 1110 used for performing processing of text strings to determine a semantic meaning of the text. To train the model 1110, a plurality of strings of text string are used by the system, where each of those strings have been labelled by a human user. Figure 10 shows a first text string 1140 labelled as indicating that the guidewire has reached a given location, a second text string 1150 labelled as being an instruction to end a current action, and a third text string 1160 labelled as being an instruction to start an injection. Each of those text strings are input by the apparatus 150 to the recurrent neural network 1110 to derive a set of outputs. For each of the text strings 1140, 1150, 1160, the outputs are compared to the corresponding label at comparison stage 1130 to determine an error/loss, which is then used to update the parameters of the model 1110.

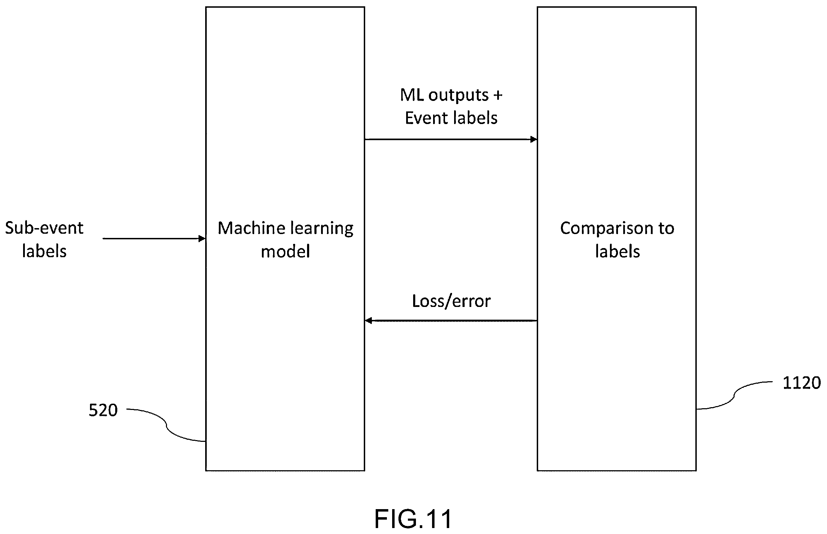

The apparatus 150 may be used to perform training of ta machine learning model 520 that may be used to derive labels for events from the sub-event labels. Reference is made to Figure 11, which illustrates a process in which a machine learning model 520 may be trained using a plurality of sub-event labels and a plurality of event labels. The event labels are provided by a human user, whereas the sub-event labels may be either provided by a human user or derived by applying the watchers 310 to different data channels as discussed above.

A set of sub-event labels is input by the apparatus 150 to the machine learning model 520 to derive output values. The output values are compared at the compare stage 1120 to one or more numerical values representing an event label provided by the human user to determine a loss/error. This loss/error is then used to output the parameters of the model 520. This process is repeated using multiple sets of sub-event labels, each having a corresponding event label.

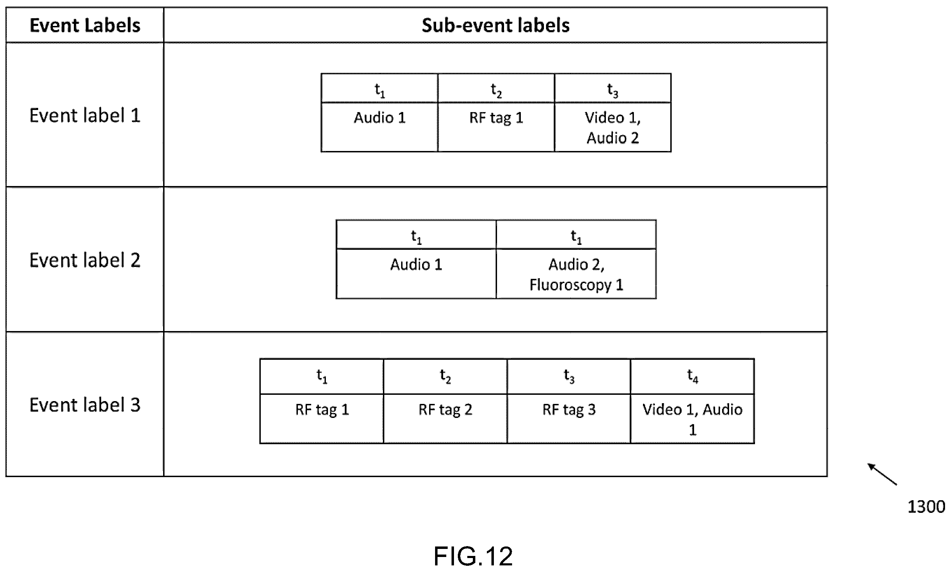

Reference is made to Figure 12, which illustrates part 1300 of an example training data set for training the machine learning model 520. The training data 1300 comprises three sets of sub-event labels, each having a corresponding event label. The first set of sub-event labels includes a label indicating a first sub-event (shown as Audio 1) that was identified in audio data at a time t1, a label indicating a second sub-event (shown as RF tag 1) that was identified by an RF detector at time t2,, a label indicating a third sub-event (shown as video 1) that was identified in video data at time t3, and a fourth sub-event (shown as audio 2) that was identified in audio data at time t3. These sub-events are each associated with an event assigned the event label 1, and are considered by a human user to be indicative of that type of event. The model 520 may be an RNN, with the first set of sub-event labels input in chronological order, starting with the earlier sub-events to derive output values from the RNN, which are compared to event label 1 at the comparison stage 1120.

The training data 1300 also comprises a second set of sub-event labels (including audio and fluoroscopy data) and a corresponding event label, and a third set of sub-event labels (including RF tag data, video data, and audio data) and a corresponding event label. The second set of sub-event labels, third set of sub-event labels and their corresponding event labels are processed to perform training of the model 520 in the same manner as the first set of sub-event labels.

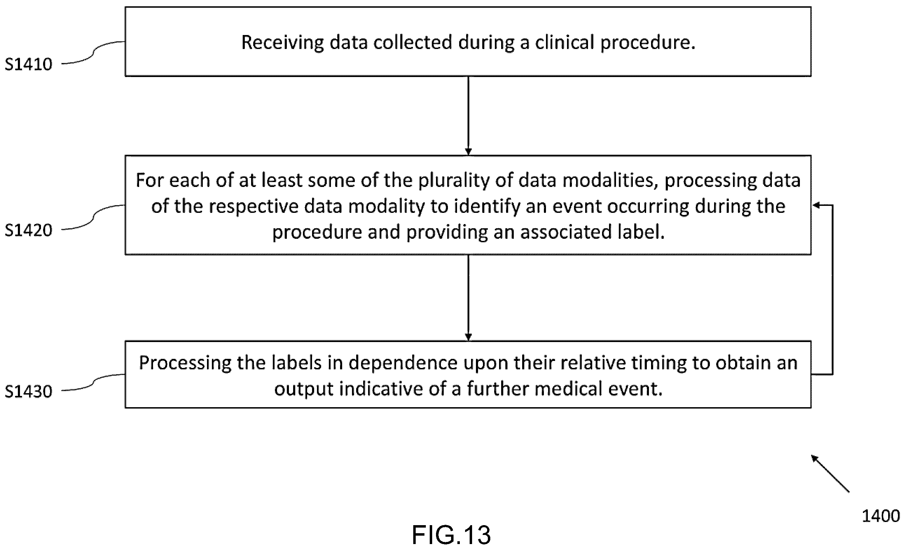

Reference is made to Figure 13, which illustrates a method 1400 implemented in the apparatus 100.

At S1410, the apparatus 100 receives data of the plurality of data modalities that is collected during the clinical procedure.

At S1420, the apparatus 100 identifies sub-events in the data of at least some of these data modalities. For each of the identified sub-events a label is produced.

At S1430, the apparatus 100 processes the labels obtained at S1420 in dependence upon their relative timing to obtain an output indicative of a further medical event, which is a higher level event than the sub-events identified at S1420. S1430 may comprise for each of the labels, upon generation of the respective label, providing the label as an input to a model 520 to obtain an output indicative of the further medical event.

The apparatus 100 may continue by performing S1420 again to derive further sub-event labels corresponding to subsequent times in the common timeline for the clinical procedure. The apparatus 100 then performs S1430 again to derive outputs indicative of another event. In some embodiments, the model 520 may be a recurrent neural network for which the output values are updated with each subsequent sub-event label input, and classifies the collection of labels as they are presented.

The process of recognising events based on data obtained from multiple channels may be performed online or offline on recorded data. In other words, the process may be performed in real-time, i.e. during a procedure, as data becomes available, or may be performed after the procedure when the full set of data belonging to the different channels has been collected. The set of categorised events can be used to create a procedure summary and report, and can be recalled individually for reference and preparation.

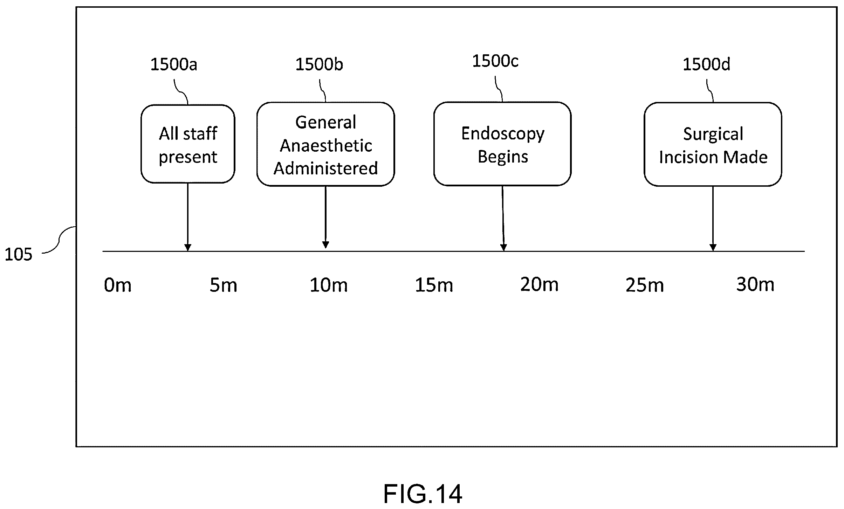

Reference is made to Figure 14, which illustrates an example of content that may be displayed on a user interface (display 105) of device 100 during or following a clinical procedure. The content includes a timeline on which a number of labels of events 1500a-d are shown. The content may be part of procedure summary and report generated following the procedure. Alternatively, the content may be part of a realtime report generating during the procedure. In the case of a realtime report, additional labels are added to the timeline in response to events detected on the basis of further collected data.

The example has been explained above in which a label is displayed on the display 105 as an output representing a medical event; however, the output is not limited to displaying. For example, the device 100 may output contents corresponding to the label representing the medical event to an analysis application. More particularly, in a scene in which a treatment is performed for a cerebral aneurysm, an output of a label representing arrival of a catheter at a given position within a cerebral blood vessel of the patient and execution of contrast radiography may trigger activation of an analysis application that measures a size of a neck (neck portion) of the cerebral aneurysm through image analysis. In this case, the device 100 may output, to the analysis application, X-ray imaging data collected through the contrast radiography. This allows a medical staff member to obtain a measurement result of the neck size of cerebral aneurysm, without performing any operation such as activation of the analysis application and input of the X-ray imaging data.

Moreover, for example, the device 100 may derive an output representing an event corresponding to a concrete order from a medical staff member, such as "Give me an image showing blood vessels well", based on the sub-event labels derived from various types of data, which have been received from various types of data collecting devices.

Implementations of the subject matter and the operations described in this specification can be realized in digital electronic circuitry, or in computer software, firmware, or hardware, including the structures disclosed in this specification and their structural equivalents, or in combinations of one or more of them. For instance, hardware may include processors, microprocessors, electronic circuitry, electronic components, integrated circuits, etc. Implementations of the subject matter described in this specification can be realized using one or more computer programs, i.e., one or more modules of computer program instructions, encoded on computer storage medium for execution by, or to control the operation of, data processing apparatus. Alternatively or in addition, the program instructions can be encoded on an artificially generated propagated signal, e.g., a machine-generated electrical, optical, or electromagnetic signal that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

According to various embodiments, there is provided an event recognition method comprising: a) receiving multiple sources of data in a synchronous manner; b) labelling salient events on individual data channels; c) passing on the labels to an event identifier algorithm; and d) identifying and labelling the event using individual channel labels accumulated over time, wherein at least one of sources of data in a) is a medical imaging data source. In some of the further embodiments, the algorithm of d) is an RNN state machine using multiple states and a multi-headed architecture. In some of the further embodiments, the algorithm of b) analyses data over discrete amount of time, and is triggered by a saliency detector on the signal. In some of the further embodiments, the identified events in d) are used to provide a summary of the procedure. In some of the further embodiments, the data in a) comprises sources of data used in a medical imaging or interventional procedure. In some of the further embodiments, the data in a) comprises one or more clinical imaging channels, video, sound, interaction with equipment, scanning of RF tags or barcode for consumable equipment. In some of the further embodiments, the method is applied to live streaming data. In some of the further embodiments, the method is applied to recorded data.

According to certain embodiments, there is provided a data processing apparatus comprising processing circuitry configured to: receive data collected during a clinical procedure, the data belonging to a plurality of data modalities, at least one of the data modalities being an imaging data type and a further of the data modalities being an additional data type other than imaging data, wherein the data is provided with reference to a common timeline over which the data is collected; for each of the plurality of data modalities, process data of the respective data modality to generate one or more labels, each identifying an event occurring at a time on the common timeline and indicated by the processed data; and process the labels for each of the identified events based on the relative time of occurrence of the events to obtain an output indicative of a further medical event.

According to certain embodiments, for each of the plurality of data modalities, the step of processing the labels for each of the identified events comprises: providing the labels as inputs to a machine learning model to obtain the output indicative of the further medical event.

According to certain embodiments, wherein for each of the data modalities, the processing of the data of the respective data modality and the generation of the label is performed in real time as the data is received, wherein providing the labels as inputs to the machine learning model comprises, for each of the labels, upon the generation of the respective label: providing the respective label as an input to the machine learning model,

According to certain embodiments, the machine learning model is a recurrent neural network.

According to certain embodiments, the processing circuitry is configured to: provide each of the labels as inputs to the machine learning model in an order in which the corresponding identified events occurred in the common timeline.

According to certain embodiments, the processing circuitry is configured to: for each of the identified events, output time information indicating a time in the common timeline at which the respective identified event occurred; and process the time information for the identified events to determine a time associated with the further medical event.

According to certain embodiments, the imaging data comprises at least one of: video data; and medical imaging data.

According to certain embodiments, the plurality of data modalities comprises one or more of: video data; audio data; medical imaging data; and radio frequency tag data.

According to certain embodiments, for one or more of the plurality of data modalities: the processing of the data of the respective data modality to identify the event occurring during the procedure comprises providing the data of the respective data modality to a further machine learning model to derive the label of the respective identified event.

According to certain embodiments, one or more of the plurality of data modalities comprises the imaging data, wherein, for the imaging data, the respective further machine learning model used to derive the label of the respective identified event comprises a convolutional neural network.

According to certain embodiments, one or more of the plurality of data modalities comprises audio data, wherein for the audio data, the respective further machine learning model used to derive the label of the respective identified event comprises a speech recognition model configured to derive text representing the audio data.

According to certain embodiments, the processing circuitry is configured to: process the text using a natural language understanding model to derive the label identifying the event.

According to certain embodiments, the processing circuitry is configured to: control a display to provide a visual display indicative of the further medical event and associated time information indicating when, in the clinical procedure, the further medical event took place.

According to certain embodiments, there is provided a method comprising: receiving data collected during a clinical procedure, the data belonging to a plurality of data modalities, at least one of the data modalities being an imaging data type and a further of the data modalities being an additional data type other than imaging data, wherein the data is provided with reference to a common timeline over which the data is collected; for each of the plurality of data modalities, processing data of the respective data modality to generate a label identifying an event occurring at a time on the common timeline and indicated by the processed data; and processing the labels for each of the identified events based on the relative time of occurrence of the events to obtain an output indicative of a further medical event.

According to certain embodiments, there is provided a computer program comprising computer readable instructions, which when executed by at least one processor, causes the at least one processor to perform a method comprising: receiving data collected during a clinical procedure, the data belonging to a plurality of data modalities, at least one of the data modalities being an imaging data type and a further of the data modalities being an additional data type other than imaging data, wherein the data is provided with reference to a common timeline over which the data is collected; for each of the plurality of data modalities, processing data of the respective data modality to generate a label identifying an event occurring at a time on the common timeline and indicated by the processed data; and processing the labels for each of the identified events based on the relative time of occurrence of the events to obtain an output indicative of a further medical event.

While certain arrangements have been described, the arrangements have been presented by way of example only, and are not intended to limit the scope of protection. The inventive concepts described herein may be implemented in a variety of other forms. In addition, various omissions, substitutions and changes to the specific implementations described herein may be made without departing from the scope of protection defined in the following claims.

## Claims

1. A medical information processing apparatus (100) comprising processing circuitry (115) configured to:
obtain a plurality of pieces of data collected during a clinical procedure, the plurality of pieces of data belonging to a plurality of data modalities (220a-220e), at least one of the plurality of data modalities (220a-220e) being an imaging data type and any other of the plurality of data modalities (220a-220e) than the imaging data type being a further data type different from imaging data, wherein the plurality of pieces of data is provided, linking with a common timeline that is common to times at which the plurality of pieces of data are collected;
for each of the plurality of data modalities (220a-220e), process data of each of the plurality of data modalities (220a-220e) to generate one or more labels occurring at a time on the common timeline and identify an event indicated by each of the processed plurality of pieces of data; and
process the labels for each of the identified events based on the relative time of occurrence of the events to output information indicative of a further medical event.

2. A medical information processing apparatus (100) as claimed in claim 1, wherein for each of the plurality of data modalities (220a-220e), the step of processing the labels for each of the identified events comprises:
providing the labels as inputs to a machine learning model (520) to obtain information indicative of the further medical event.

3. A medical information processing apparatus (100) as claimed in claim 2, wherein for each of the data modalities (220a-220e), the processing of the data of each of the plurality of data modalities (220a-220e) and the generation of the label is performed in real time when one of the plurality of pieces of data is obtained, wherein providing the labels as inputs to the machine learning model (520) comprises, for each of the labels, upon the generation of the respective label:
providing the respective label as an input to the machine learning model (520).

4. A medical information processing apparatus (100) as claimed in claim 2 or claim 3, wherein the machine learning model (520) is a recurrent neural network.

5. A medical information processing apparatus (100) as claimed in claim 2 or 3, wherein the processing circuitry (115) is configured to:
provide each of the labels as inputs to the machine learning model (520) in an order in which the corresponding identified events occurred in the common timeline.

6. A medical information processing apparatus (100) as claimed in claim 2 or 3, wherein the processing circuitry (115) is configured to:
for each of the identified events, output time information indicating a time in the common timeline at which the respective identified event occurred; and
process the time information for the identified events to determine a time associated with the further medical event.

7. A medical information processing apparatus (100) as claimed in claim 2 or 3, wherein the imaging data comprises at least one of:
video data;
X-ray image data; and
ultrasonic image data.

8. A medical information processing apparatus (100) as claimed in claim 2 or 3, wherein the plurality of data modalities (220a-220e) comprises at least one of:
video data;
audio data;
X-ray image data;
ultrasonic image data;
radio frequency tag data; and
vital data.

9. A medical information processing apparatus (100) as claimed in claim 2 or 3, wherein for one or more of the plurality of data modalities (220a-220e):
the processing of the data of each of the plurality of data modalities (220a-220e) to identify the event occurring during the procedure comprises providing the data of each of the data modalities (220a-220e) to a further machine learning model (520) to derive the label of the respective identified event.

10. A medical information processing apparatus (100) as claimed in claim 9, wherein one or more of the plurality of data modalities (220a-220e) comprises the imaging data,
wherein, for the imaging data, the respective further machine learning model (520) used to derive the label of the respective identified event comprises a convolutional neural network.

11. A medical information processing apparatus (100) as claimed in claim 9 or claim 10, wherein one or more of the plurality of data modalities (220a-220e) comprises audio data,
wherein for the audio data, the respective further machine learning model used to derive the label of the respective identified event comprises a speech recognition model configured to derive text representing the audio data.

12. A medical information processing apparatus (100) as claimed in claim 11, wherein the processing circuitry (115) is configured to:
process the text using a natural language understanding model to derive the label identifying the event.

13. A medical information processing apparatus (100) as claimed in claim 2 or 3, wherein the processing circuitry (115) is configured to:
control a display (105) to display on the display (105) visual information indicative of the further medical event and associated time information indicating when, in the clinical procedure, the further medical event took place.

14. A medical information processing apparatus (100) as claimed in claim 2 or 3, wherein the processing circuitry (115) is configured to:
execute an application corresponding to the further medical event in accordance with an output of information indicative of the further medical event.

15. A medical information processing method performed by a medical information processing apparatus (100), the method comprising:
obtaining a plurality of pieces of data collected during a clinical procedure, the plurality of pieces of data belonging to a plurality of data modalities (220a-220e), at least one of the plurality of data modalities (220a-220e) being an imaging data type and any other of the plurality of data modalities (220a-220e) than the imaging data type being a further data type different from imaging data, wherein the plurality of pieces of data is provided, linking with a common timeline that is common to times at which the plurality of pieces of data are collected;
for each of the plurality of data modalities (220a-220e), processing data of each of the plurality of data modalities (220a-220e) to generate one or more labels occurring at a time on the common timeline and identifying an event indicated by each of the processed plurality of pieces of data; and
processing the labels for each of the identified events based on the relative time of occurrence of the events to output information indicative of a further medical event.
